(19) Europäisches Patentamt / European Patent Office / Office européen des brevets

(11) **EP 3 658 021 B1**

(12) **EUROPEAN PATENT SPECIFICATION**

(45) Date of publication and mention
of the grant of the patent:
**07.02.2024 Bulletin 2024/06**

(21) Application number: **18839161.9**

(22) Date of filing: **26.07.2018**

(51) International Patent Classification (IPC):
**A61B 5/085** *(2006.01)*    **A61B 5/087** *(2006.01)*
**A61B 5/091** *(2006.01)*

(52) Cooperative Patent Classification (CPC):
**A61B 5/085; A61B 5/087; A61B 5/091**

(86) International application number:
**PCT/CA2018/050908**

(87) International publication number:
**WO 2019/018938 (31.01.2019 Gazette 2019/05)**

(54) **METHOD AND SYSTEM TO ACQUIRE OSCILLOMETRY MEASUREMENTS**

VERFAHREN UND SYSTEM ZUR ERFASSUNG VON OSZILLOMETRISCHEN MESSUNGEN

PROCÉDÉ ET SYSTÈME POUR ACQUÉRIR DES MESURES D'OSCILLOMÉTRIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.07.2017 US 201762537228 P**

(43) Date of publication of application:
**03.06.2020 Bulletin 2020/23**

(73) Proprietor: **Thorasys Thoracic Medical Systems
Inc.
Montréal, Québec H2V 4L5 (CA)**

(72) Inventors:
• **SCHUESSLER, Thomas F.
Montréal
Québec H2W 2L4 (CA)**
• **DRAPEAU, Guy
Montréal
Québec H2V 4L5 (CA)**
• **MAKSYM, Geoffrey N.
Dartmouth
Nova Scotia B2Y 2E9 (CA)**

(74) Representative: **Rummler, Felix et al
Maucher Jenkins
Liebigstrasse 39
80538 München (DE)**

(56) References cited:
**WO-A1-2013/177621    US-A- 6 142 952**

US-A1- 2012 289 852    US-A1- 2015 119 743
US-B1- 6 675 797    US-B1- 6 675 797
US-B2- 7 267 652

• **Alamer Marwa: "Repeatability Of Respiratory
Impedance And Bronchodilatory Response In
Asthmatic Children", , 1 March 2016 (2016-03-01),
pages 1-93, XP055784302, Dalhousie University
(Faculty of Graduate Studies Online Theses),
Halifax, Nova Scotia Retrieved from the Internet:
URL:https://dalspace.library.dal.ca/handle
/10222/71242 [retrieved on 2021-03-10]**
• **SHARSHAR et al.: "The utility of impulse
oscillometry in asthma: A comparison of
spirometry versus impulse oscillometry system",
Egyptian Journal of Chest Diseases and
Tuberculosis, vol. 66 18 March 2017 (2017-03-18),
pages 207-209, XP055566607, Retrieved from the
Internet:
URL:http://dx.doi.org/10.1016/j.ejcdt.2017 .03.00
2**
• **MERAZ et al.: "Analysis of impulse oscillometric
measures of lung function and respiratory
system model parameters in small
airway-impaired and healthy children over a
2-year period", BioMedical Engineering, vol. 10,
no. 21 25 March 2011 (2011-03-25), pages 1-21,
XP021096801, Retrieved from the Internet:
URL:http://www.biomedical-engineering-onli
ne.com/content/10/1/21**

- MERAZ et al.: "Reference Equations for Impulse Oscillometric and Respiratory System Model Parameters in Anglo and Hispanic Children", Revista Mexican a de Ingenieria Biomedica, vol. 37, no. 1 pages 49-61, XP055566785, Retrieved from the Internet: URL:https://pdfs.semanticscholar.org/d20f/ 3c2f101a5bc68b6453b688d4ac5393fe8ca2.pdf
- LEHMAN et al.: "Methods of Blood Pressure Measurement in the ICU", NIH Public Access Author Manuscript, vol. 41, no. 1 January 2013 (2013-01), pages 1-13, XP055566787, Retrieved from the Internet: URL:https://www.ncbi.nlm.nih.gov/pmc/artic les/PMC3724452
- KOOHI: "Methods for Non-invasive Trustworthy Estimation of Arterial Blood Pressure", Electrical Engineering and Computer Science - Theses, 16 February 2017 (2017-02-16), pages 1-4, XP055671660, Retrieved from the Internet: URL:http://hdl.handle.net/10393/35830
- Schulz Holger ET AL: "Reference Values of Impulse Oscillometric Lung Function Indices in Adults of Advanced Age", PLoS ONE, vol. 8, no. 5, 15 May 2013 (2013-05-15), page e63366, XP093010677, DOI: 10.1371/journal.pone.0063366
- Guo Y. F. ET AL: "Normal values for respiratory resistance using forced oscillation in subjects >65 years old", EUROPEAN RESPIRATORY JOURNAL, vol. 26, no. 4, 1 October 2005 (2005-10-01), pages 602-608, XP093010682, GB ISSN: 0903-1936, DOI: 10.1183/09031936.05.00010405 Retrieved from the Internet: URL:https://erj.ersjournals.com/content/er j/26/4/602.full.pdf>

**Description**

CROSS-REFERENCE TO RELATED APPLICATIONS

The present application claims priority on United States Patent Application No. 62/537,228 filed July 26, 2017.

TECHNICAL FIELD

The present application relates to oscillometry measurements, for instance in the context of clinical pulmonary function testing.

BACKGROUND OF THE ART

In clinical pulmonary function testing, it is common practice to obtain at least three repetitions of a given measurement. For lung function assessment by oscillometry (also known as the Forced Oscillation Technique, FOT), it is commonly known to acquire a minimum of three independent, valid measurements, such that the final result is calculated as the average of such three measurements. To avoid outlier measurements, the coefficient of variation (CV) between the three measurements included in the average should be less than a set threshold or else such tests are discarded.

Current oscillometry systems operated with such a procedure may therefore require a human operator to (i) assess a reading of variability such as a CV; (ii) take a decision about whether the measurements obtained are sufficiently reproducible or if further measurements need to be acquired; and (iii) select which measurements to include in or exclude from the average if additional measurements have been acquired. As a result, different criteria may be applied depending on operator skill and preferences. For example, operators have the choice to examine CVs for respiratory system resistance (R), reactance (X) or impedance (Z), each at a single frequency only or across the entire range of frequencies measured.

Using conventional techniques, additional variability may be introduced by the fact that each measurement may be subjected to varying degrees of individual quality control and breath segmentation before it is combined with other measurements to calculate average and CV. Considering a scenario in which three individual measurements of equal duration are collected, it is possible that the breath boundaries are positioned differently within the measurement such that one measurement contains one less complete breath than another. If, in addition, that measurement contains an artefact that leads to the removal of another breath, it is conceivable that the number of complete breaths contained in the three measurements contained in a given set differs by two or more breaths. Accordingly, these measurements contain different amounts of valid data, so that they no longer become comparable items for averaging. In such a scenario it would make sense to either isolate comparable episodes such as breaths, and to average and calculate CVs across such episodes rather than across measurements, or to control the system such that the likelihood of acquiring an equal number of breaths is increased, or both.

It would therefore be desirable to standardize and automate the acquisition of oscillometry measurements in order to facilitate test execution and reduce operator dependence of outcomes.

US 2012/289852 A1 describes a method of estimating respiratory impedance, the method comprising: coupling a patient interface device to an airway of a subject to create a pneumatic system that includes the patient interface device and an airway of such a subject; oscillating pressure, flow, or volume of gas in such an airway of such a subject via an excitation source operatively coupled to the patient interface device; determining the flow and pressure of the gas in the pneumatic system to produce a respective time series representing the flow and the pressure; transforming the respective time series to the time-frequency domain to create a transformed time series; estimating the power of the flow and the pressure from the transformed time series; estimating respective cross spectra of the flow and the pressure based the transformed time series; and estimating the respiratory impedance of the subject from the estimated power and the estimated cross spectra.

US 6 142 952 describes a method of determining the airway impedance of a patient comprising the steps of: supplying breathable gas to the patient's airways with an interface; applying a selected audio-frequency oscillation component to a pressure of the breathable gas; measuring pressure and flow of the breathable gas in the interface to obtain pressure and flow data characteristic of the patient's airway; converting the pressure and flow data to the frequency domain; and obtaining a mathematical function of the processed pressure and flow data to determine an index of airway impedance or degree of airway obstruction.

SUMMARY

Therefore, it is an aim of the present disclosure to provide a method to acquire oscillometry measurements that addresses issues associated with the prior art.

It is a further aim of the present disclosure to provide a system to acquire oscillometry measurements that addresses issues associated with the prior art.

The present invention is defined in the independent claims. The dependent claims recite selected optional features.

In the following, each of the described methods, apparatuses, examples, and aspects, which do not fully correspond to the invention as defined in the claims is thus not according to the invention and is, as well as the whole following description, present for illustration purposes only or to highlight specific aspects or features of the claims.

In accordance with an embodiment of the present disclosure, there is provided a method for acquiring oscillometry measurements with an oscillometry measuring system comprising: receiving oscillometry measurements, using at least one processor of the oscillometry measuring system, the oscillometry measurements being from at least one oscillometry recording; identifying, using the at least one processor of the oscillometry measuring system, parameters in the oscillometry measurements; calculating, using the at least one processor of the oscillometry measuring system, at least one objective function from the parameters of the oscillometry measurements, evaluating, using the at least one processor of the oscillometry measuring system, the at least one objective function as a function of at least one predetermined threshold; accepting or rejecting, using the at least one processor of the oscillometry measuring system, the oscillometry measurements from the evaluating; and outputting, using the at least one processor of the oscillometry measuring system, oscillometry data using the oscillometry measurements if accepted from the evaluating.

In the method as described herein, receiving oscillometry measurements may further include receiving oscillometry measurements delimited by breathing episodes.

In the method as described herein, receiving oscillometry measurements delimited by breathing episodes may include isolating the breathing episodes from the at least one oscillometry recording.

In the method as described herein, isolating the breathing episodes may include identifying a marker in the at least one oscillometry recording including at least one of maxima, minima, zero crossings, and crossings of pre-defined threshold values of flow or volume signals.

In the method as described herein, receiving oscillometry measurements delimited by breathing episodes may include monitoring a breath of a subject and triggering a recording of the oscillometry measurements upon detection of a desired point in a breathing cycle.

In the method as described herein, triggering a recording of the oscillometry measurements may include identifying a marker in the monitoring including at least one of maxima, minima, zero crossings, and crossings of predefined threshold values of flow or volume signals.

In the method as described herein, receiving oscillometry measurements may include recording the oscillometry measurements during the at least one oscillometry recording using an oscillometry measurement device from the at least one oscillometry measurement system.

In the method as described herein, identifying the parameters in the oscillometry measurements may include identifying at least one of respiratory system resistance, reactance or impedance.

In the method as described herein, calculating at least one objective function may include calculating a coefficient of variation (CV) of the resistance at a single frequency f* according to $\zeta = CV(R(f*))$.

In the method as described herein, calculating at least one objective function may include calculating a coefficient of variation (CV) of impedance at a single frequency f* according to $\zeta = CV(|Z(f*)|)$.

In the method as described herein, calculating at least one objective function may include calculating a maximum coefficient of variation (CV) of a resistance over a range of frequencies according to $\zeta = max(CV(R(f)))$.

In the method as described herein, calculating at least one objective function may include calculating a maximum coefficient of variation (CV) of an impedance over a range of frequencies according to $\zeta = max(CV(|Z(f)|))$.

In the method as described herein, calculating at least one objective function may include calculating an average of coefficients of variation (CV) of an impedance over Nf frequencies measured according to

$$\zeta = \frac{1}{N_f} \sum_{i=1}^{N_f} CV\left(\left|z_{f_i}\right|\right).$$

In the method as described herein, calculating at least one objective function may include calculating said average of coefficients of variation (CV) of the impedance over Nf frequencies by adding a weighing function W according to

$$\zeta = \frac{\sum_{i=1}^{N_f} W_i \cdot CV\left(\left|Z_{f_i}\right|\right)}{\sum_{i=1}^{N_f} W_i}.$$

In the method as described herein, calculating at least one objective function may include calculating said average of coefficients of variation (CV) of the impedance over Nf frequencies by calculating a root mean squared value according to

$$\zeta = \left( \frac{\sum_{i=1}^{Nf} W_i \cdot CV(|Z_{f_i}|)^2}{\sum_{i=1}^{Nf} W_i} \right)^{\frac{1}{2}}.$$

In the method as described herein, calculating at least one objective function may include calculating said average of coefficients of variation (CV) of the impedance over Nf frequencies by calculating a root mean squared value in which an order of the root marches a power p to which each CV is elevated to according to

$$\zeta = \left( \frac{\sum_{i=1}^{Nf} W_i \cdot CV(|Z_{f_i}|)^p}{\sum_{i=1}^{Nf} W_i} \right)^{\frac{1}{p}}.$$

In the method as described herein, calculating at least one objective function may include calculating a sum of squared standard deviations divided by a sum of squared means of |Z| across the Nf frequencies measured according to

$$\zeta = \frac{\sum_{i=1}^{Nf} SD(|Z_{f_i}|)^2}{\sum_{i=1}^{Nf} \overline{Z_{f_i}}^2}.$$

In the method as described herein, calculating at least one objective function may include calculating a sum of squared standard deviations divided by a sum of squared means of |Z| across the Nf frequencies measured, with an added weighing function W according to

$$\zeta = \frac{\sum_{i=1}^{Nf} W_i \cdot SD(|Z_{f_i}|)^2}{\sum_{i=1}^{Nf} W_i \cdot \overline{|Z_{f_i}|}^2}.$$

In the method as described herein, calculating at least one objective function may include calculating a sum of standard deviations elevated to a power p divided by a sum of means of |Z| elevated to the power p, across Nf frequencies measured, including a weighing function W according to

$$\zeta = \frac{\sum_{i=1}^{Nf} W_i \cdot SD(|Z_{f_i}|)^p}{\sum_{i=1}^{Nf} W_i \cdot \overline{|Z_{f_i}|}^p}.$$

In the method as described herein, calculating at least one objective function may include determining if a minimum number $N_{min}$ of oscillometry measurements is reached prior to calculating the at least one objective function.

The method as described herein may further comprise combining oscillometry measurements if the minimum number $N_{min}$ of oscillometry measurements is exceeded, the combining include all permutations having at least a minimum number $N_{av}$ of oscillometry measurements required for averaging.

In the method as described herein, evaluating the at least one objective function may include evaluating the at least one objective function using one of the permutations selected as a function of the at least one predetermined threshold.

In the method as described herein, calculating at least one objective function may include calculating the objective function using an average of the parameters for the oscillometry measurements.

In the method as described herein, accepting or rejecting the oscillometry measurements may include rejecting the oscillometry measurements if a maximum number $N_{max}$ of oscillometry measurements is exceeded.

In the method as described herein, evaluating the oscillometry measurements may include grading the oscillometry measurements as a function of the at least one predetermined threshold.

In accordance with another embodiment of the present disclosure, there is also provided a system for acquiring oscillometry measurements comprising: a processing unit; and a non-transitory computer-readable memory communicatively coupled to the processing unit and comprising computer-readable program instructions executable by the processing unit for: receiving oscillometry measurements, using at least one processor of the oscillometry measuring system, the oscillometry measurements being from at least one oscillometry recording; identifying, using the at least one processor of the oscillometry measuring system, parameters in the oscillometry measurements; calculating, using the at least one processor of the oscillometry measuring system, at least one objective function from the parameters of the oscillometry measurements, evaluating, using the at least one processor of the oscillometry measuring system, the at least one objective function as a function of at least one predetermined threshold; accepting or rejecting, using the at least one processor of the oscillometry measuring system, the oscillometry measurements from the evaluating; and outputting, using the at least one processor of the oscillometry measuring system, oscillometry data using the oscillometry measurements if accepted from the evaluating.

In the system as described herein, receiving oscillometry measurements may include receiving oscillometry measurements delimited by breathing episodes.

In the system as described herein, receiving oscillometry measurements delimited by breathing episodes may include isolating the breathing episodes from the at least one oscillometry recording.

In the system as described herein, isolating the breathing episodes may include identifying a marker in the at least one oscillometry recording including at least one of maxima, minima, zero crossings, crossings of pre-defined threshold values of flow or volume signals.

In the system as described herein, receiving oscillometry measurements delimited by breathing episodes may include monitoring a breath of a subject and triggering a recording of the oscillometry measurements upon detection of a desired point in a breathing cycle.

In the system as described herein, triggering a recording of the oscillometry measurements may include identifying a marker in the monitoring including at least one of maxima, minima, zero crossings, crossings of pre-defined threshold values of flow or volume signals.

In the system as described herein, receiving oscillometry measurements may include recording the oscillometry measurements during the at least one oscillometry recording using an oscillometry measurement device from the at least one oscillometry measurement system.

In the system as described herein, identifying the parameters in the oscillometry measurements may include identifying at least one of respiratory system resistance, reactance or impedance.

In the system as described herein, calculating at least one objective function may include calculating a coefficient of variation (CV) of the resistance at a single frequency f* according to $\zeta = CV( R( f^* ))$.

In the system as described herein, calculating at least one objective function may include calculating a coefficient of variation (CV) of impedance at a single frequency f* according to $\zeta = CV( |Z( f^* )| )$.

In the system as described herein, calculating at least one objective function may include calculating a maximum coefficient of variation (CV) of a resistance over a range of frequencies according to $\zeta = \max( CV( R( f )))$.

In the system as described herein, calculating at least one objective function may include calculating a maximum coefficient of variation (CV) of an impedance over a range of frequencies according to $\zeta = \max( CV(|Z(f)|))$.

In the system as described herein, calculating at least one objective function may include calculating an average of coefficients of variation (CV) of an impedance over $N_f$ frequencies measured according to

$$\zeta = \frac{1}{N_f} \sum_{i=1}^{N_f} CV\left(\left|z_{f_i}\right|\right)$$

In the system as described herein, calculating at least one objective function may include calculating said average of coefficients of variation (CV) of the impedance over $N_f$ frequencies by adding a weighing function W according to

$$\zeta = \frac{\sum_{i=1}^{N_f} W_i \cdot CV\left(\left|Z_{f_i}\right|\right)}{\sum_{i=1}^{N_f} W_i}.$$

In the system as described herein, calculating at least one objective function may include calculating said average of coefficients of variation (CV) of the impedance over $N_f$ frequencies by calculating a root mean squared value according to

$$\zeta = \left( \frac{\sum_{i=1}^{N_f} W_i \cdot CV(|z_{f_i}|)^2}{\sum_{i=1}^{N_f} W_i} \right)^{\frac{1}{2}}.$$

In the system as described herein, calculating at least one objective function may include calculating said average of coefficients of variation (CV) of the impedance over $N_f$ frequencies by calculating a root mean squared value in which an order of the root marches a power p to which each CV is elevated to according to

$$\zeta = \left( \frac{\sum_{i=1}^{N_f} W_i \cdot CV(|z_{f_i}|)^p}{\sum_{i=1}^{N_f} W_i} \right)^{\frac{1}{p}}.$$

In the system as described herein, calculating at least one objective function may include calculating a sum of squared standard deviations divided by a sum of squared means of |Z| across the $N_f$ frequencies measured according to

$$\zeta = \frac{\sum_{i=1}^{N_f} SD(|z_{f_i}|)^2}{\sum_{i=1}^{N_f} \overline{z_{f_i}}^2}.$$

In the system as described herein, calculating at least one objective function may include calculating a sum of squared standard deviations divided by a sum of squared means of |Z| across the $N_f$ frequencies measured, with an added weighing function W according to

$$\zeta = \frac{\sum_{i=1}^{N_f} W_i \cdot SD(|z_{f_i}|)^2}{\sum_{i=1}^{N_f} W_i \cdot \overline{|z_{f_i}|}^2}.$$

In the system as described herein, calculating at least one objective function may include calculating a sum of standard deviations elevated to a power p divided by a sum of means of |Z| elevated to the power p, across Nf frequencies measured, including a weighing function W according to

$$\zeta = \frac{\sum_{i=1}^{N_f} W_i \cdot SD(|z_{f_i}|)^p}{\sum_{i=1}^{N_f} W_i \cdot \overline{|z_{f_i}|}^p}.$$

In the system as described herein, calculating at least one objective function may include determining if a minimum number $N_{min}$ of oscillometry measurements is reached prior to calculating the at least one objective function.

The system as described herein may further comprise combining oscillometry measurements if the minimum number $N_{min}$ of oscillometry measurements is exceeded, the combining include all permutations having at least a minimum number $N_{av}$ of oscillometry measurements required for averaging.

In the system as described herein, evaluating the at least one objective function may include evaluating the at least one objective function using one of the permutations selected as a function of the at least one predetermined threshold.

In the system as described herein, calculating at least one objective function may include calculating the objective function using an average of the parameters for the oscillometry measurements.

In the system as described herein, accepting or rejecting the oscillometry measurements may include rejecting the oscillometry measurements if a maximum number $N_{max}$ of oscillometry measurements is exceeded.

In the system as described herein, evaluating the oscillometry measurements may include grading the oscillometry measurements as a function of the at least one predetermined threshold.

The system as described herein may further comprise the oscillometry device for recording the at least one oscillometry recording.

In accordance with a further embodiment of the present disclosure, there is also provided a system for acquiring oscillometry measurements comprising: an acquisition module configured for receiving repeated oscillometry measurements from a device and identifying parameters from the oscillometry measurements; and an objective function evaluator module calculating at least one objective function from the parameters of the repeated oscillometry measurements, and evaluating the at least one objective function as a function of at least one predetermined threshold, the objective function evaluator module accepting or rejecting oscillometry measurements from the evaluating; whereby the system outputs oscillometry data using accepted oscillometry measurements from the evaluating.

DESCRIPTION OF THE DRAWINGS

Fig. 1 is a block diagram of a system to acquire oscillometry measurements in accordance with the present disclosure;

Fig. 2 is a flowchart of a method to assess acquired oscillometry measurements in accordance with the present disclosure;

Fig. 3 is a flowchart of an extended method to assess acquired oscillometry measurements in accordance with the present disclosure, with grading,

Fig. 4 is a flowchart of an extended method to assess episodes such as breaths isolated from acquired oscillometry measurements in accordance with the present disclosure;

Fig. 5 is a flowchart of an extended method to assess acquired oscillometry measurements in accordance with the present disclosure, wherein measurement acquisition is controlled such that the onset of all measurements coincides with the same point in the breathing cycle and/or occurs if conditions to confirm stability of the breathing pattern have been met;

Fig. 6 shows exemplary average coefficients of variation from variable patients using no qualification, manual qualification by an expert and automatic qualification as per a method of the present disclosure;

Fig. 7 shows exemplary individual coefficients of variation from variable patients using no qualification, manual qualification by an expert and automatic qualification as per a method of the present disclosure; and

Fig. 8 is a schematic view of an exemplary oscillometry measurement device that may be used with the methods and systems described herein.

DETAILED DESCRIPTION

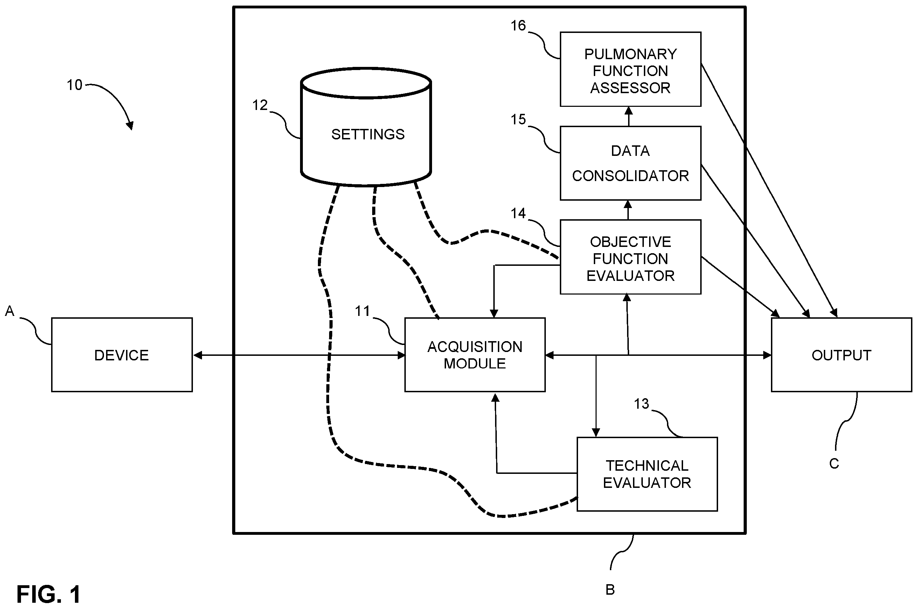

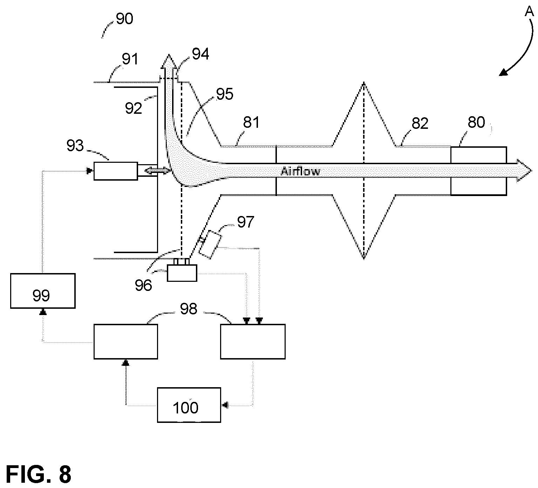

Referring to the drawings, and more particularly to Fig. 1, there is illustrated a system 10 to acquire oscillometry measurements in accordance with the present disclosure, in such a way that the acquired oscillometry measurements comply with desired acquisition parameters. The system 10 may then use the oscillometry measurements to assess the pulmonary mechanics of a patient. The system 10 is illustrated as having an oscillometry measurement device A that typically consists of at least a breathing pathway with a patient port and an atmosphere port, an oscillator adding an oscillatory component to the patient's breathing, and a flow meter measuring airflow in and out of the patient. For example, the oscillator may be a loudspeaker, a mechanical ventilator capable of producing oscillatory flows, a vibrating mesh in the flow pathway or a piezoelectric beam bending actuated devices among possible oscillators. The flow meter may be a conventional screen a pneumotachograph, an ultrasonic flow meter, a wave tube or a variable orifice flow meter, to name a few options among others. The device A may be known as an oscillometry apparatus or system, an airwave oscillometry apparatus or system, an impulse oscillometry apparatus or system, a forced-oscillation technique (FOT) apparatus or system, and/or a forced oscillometry apparatus or system, among possible names. A non-limitative embodiment of an oscillometry measurement device that may be used with the methods and systems of the present disclosure is described hereinafter with reference to Fig. 8. The device A is used in conjunction with an oscillometry acquisition processor B that produces an output C in any appropriate format. For example, the output C may be a monitor, a data file, a parameter, a set of parameters, a signal, a table, a graph, a chart or a report, each providing data quantifying the oscillometry measurements, or an average, standard deviation, median, maximum, minimum or similar consolidated measure thereof, and in some instances assessing pulmonary mechanics, or used subsequently to assess or assist in assessing pulmonary mechanics. Although the device A, the processor B and the output C are shown in Fig. 1 as being discrete components, the processor B and output C may be integrated to the device A. Processor B and output C may also be connected in any appropriate way to the device A, and may for instance be embodied as a computer, a tablet, etc. The processor B may be part of one or more computers, and may include multiple processor units as well. However, for simplicity, reference is made herein to a processor B. The processor B must have sufficient computing speed to receive and interpret data produced by the device A *in situ,* for example in real-time or quasi-real time. Moreover, the processor B, as described below, must perform tasks with the acquired oscillometry measurements to determine if it meets testing requirements. In these circumstances, the processor B must perform the tasks *in situ,* in the ongoing clinical session, and often between oscillometry measurements, as a rejected measurement may influence the testing protocol. Accordingly, not only are objective functions and grading schemes calculated by the processor B, as described herein, but the determined objective functions and grading schemes are used to adjust the workflow and prompt users to collect additional recordings if - and only if - they are needed. Thus, the processor B performs, during an ongoing clinical testing session, the determination as to whether or not each acquired oscillometry measurement meets the predetermined testing requirements or not, and can advise the user accordingly - for example after each acquired measurement - and then automatically adjust the workflow accordingly. Due to the fact that the testing protocol may include the evaluation of multiple objective functions using signal interpretation from the device A, and real time analysis as to the suitability of each newly acquirement measurement relative to the determined testing requirements, appropriate computing capacity is required for the processor B.

The processor B has an acquisition module 11 that is programmed to perform oscillometry measurement testing. The acquisition module 11 activates and controls the device A, receives raw data signals from the device A, and may convert them to an initial set of useful oscillometry measurement data, identifying and/or calculating measurement parameters that may include respiratory system resistance, reactance or impedance, to name a few options. The acquisition module 11 may also segment the measurements into periodic episodes such as breaths, a measurement being constituted typically of one or more complete episodes. To clarify, the system 10 and methods described herein performing patient testing, by which one or more oscillometry recordings are performed. An oscillometry recording may be generally defined as the moment extending from the start to the end of one contiguous session of recording sensor data for the patient using the device A. Accordingly, an oscillometry recording may include one or more breathing cycles (i.e., breaths), a breath also referred to as an episode. Episodes may also be defined by segments of a breath, such as the inspiratory phase and the expiratory phase of a breath, or singular points or regions of a breath, such as end-inspiration or end-expiration. For the purposes of the present disclosure, an oscillometry recording may include one or more oscillometry measurements, as the oscillometry measurement is an episode or a full recording, or segments of a full recording, that contains the necessary data to calculate a cost function representative of pulmonary function. Accordingly, the expression "oscillometry measurement" is used herein to include one or more episodes of breathing, a full oscillometry recording, and/or segments of a full oscillometry recording. The expression "breathing episodes" is intended to include full breaths, segments of breaths, and/or recordings of same.

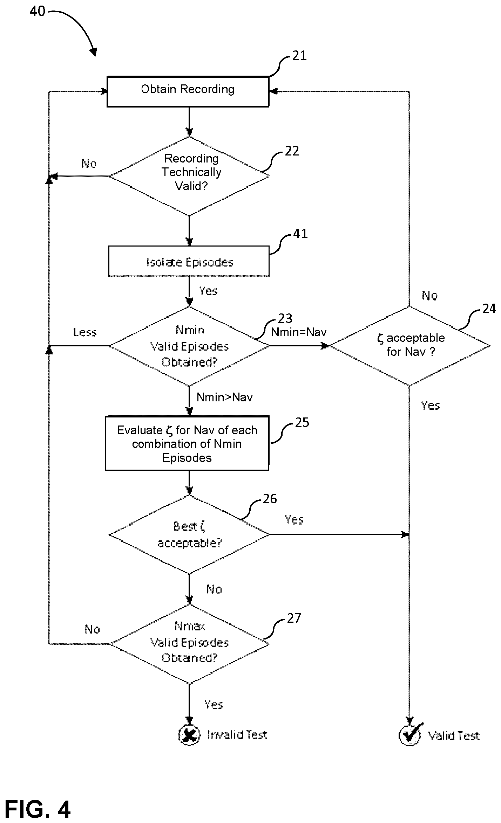

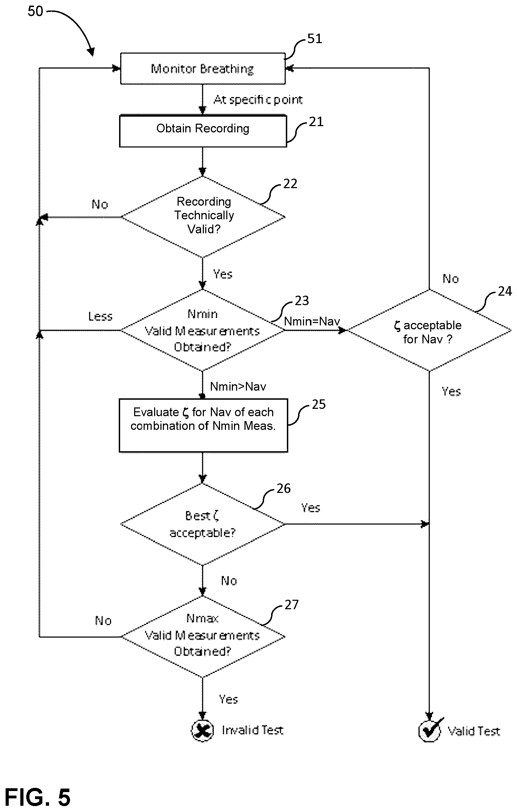

The acquisition module 11 may have the capacity to acquire live streams of breathing pressure and/or flow data immediately prior to an oscillometry recording, and may have the capacity to segment such pre-recording data streams to isolate episodes of breathing, in order to control the device A or delimit measurements such that the onset of a recording (i) always coincides with the same point in the breathing cycle, and/or (ii) occurs only if conditions to confirm stability of the breathing pattern have been met. The steps performed by the acquisition module 11 are described in further detail below, with reference to Figs. 4 and 5 for example. The acquisition module 11 may also drive the testing via the output C by displaying information on how the testing should be conducted. For this purpose, the acquisition module 11 may have access to a settings database 12, shown as being an integral part of the processor B, but alternatively accessible remotely. In fact, various modules of the processor B may be cloud-based, and accessible via telecommunications, as one possible embodiment in addition to one in which the modules are integrated in the processor B. The settings database 12 may also include settings controlling the position of the onset of a measurement within the breathing cycle, the segmentation of a measurement into episodes such as breaths, the evaluation of objective function(s) ($\zeta$), acceptance criteria for $\zeta$, and other settings such as the number of oscillometry measurements to be averaged ($N_{av}$), the minimum number of oscillometry measurements or episodes required ($N_{min}$) and the maximum number of oscillometry measurements or episodes permitted ($N_{max}$), all of which may be related to ensuring the oscillometry testing is properly and repeatably conducted and meets standards for being used to properly assess pulmonary function. The settings are described in further details hereinafter.

The acquisition module 11 may also operate with a technical evaluator module 13 that is configured to evaluate whether the individual oscillometry measurements or episodes are technically valid. For example, factors that may influence the technical validity include duration, clipping of raw data, insufficient magnitude of breathing or oscillatory pressure, flow or volume waveforms, breathing irregularities, insufficient number of breaths, poor mathematical indicators such as coherence or signal-to-noise ratio, artefacts such as leaks, coughs or swallowing detected, and insufficient remaining data volume after exclusion of such artefacts. Thus, during the monitoring phase 51 (see Fig. 5), the technical evaluator module 13 is operable to confirm the stability of the breathing pattern, for example by determining whether a set number of repeatable breaths (e.g. three consecutive breaths with comparable inspiratory time, expiratory time, tidal volume, peak flow, and/or flow shape index, etc.) before triggering a measurement capture. The technical evaluator module 13 monitors these factors and may indicate if any given oscillometry measurement or episode is, by itself, technically valid. Based on this result, the acquisition module 11 may count the number of oscillometry measurements or episodes taken, as the count value of number of measurements or episodes is used in determining if the testing is valid or has failed, as discussed hereinafter. In an embodiment, the system 10 is without the technical evaluator module 13.

An objective function evaluator module 14 receives the data of valid oscillometry measurements or episodes, which may include raw data as well as any output produced by the acquisition module 11, and performs the function of determining if a combination of multiple oscillometry measurements or episodes constitutes a valid test. Accordingly, the objective function evaluator module 14 is programmed or accesses the objective functions that are identified as being relevant to this determination, and has the algorithms necessary to calculate the objection function values from the oscillometry data. The objective functions are also detailed hereinafter. The objective function evaluator module 14 may evaluate one or more of the objective functions, and may grade the test based on the objective function(s)

if the objective function evaluator module 14 determines that the oscillometry recordings and/or oscillometry measurements meet applicable test standards and thus constitute a valid test, selected oscillometry measurements are output via the output C. The selected oscillometry measurements may be used for calculations to an appropriate value, such as an average, standard deviation, median, maximum, minimum or similar consolidated measure, by the data consolidation module 15. Moreover, using a pulmonary function assessment module 16, the processor B may also perform further analysis of the consolidated measures provided by data consolidation module 15 in order to further assist in or facilitate the analysis and interpretation of the data and the assessment of pulmonary function. Accordingly, the output C may display or output the data from any one of the acquisition module 11, the objective function evaluator module 14, data consolidation module 15, and/or the pulmonary function assessment module 16.

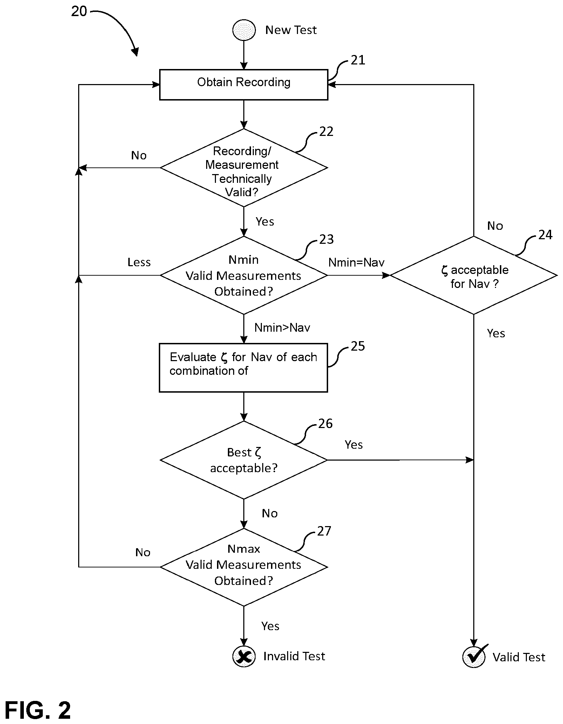

The system 10 may operate using a method to acquire oscillometry measurements, as shown at 20 in Fig. 2. According to the invention, the method 20 requires:

- at least one objective function ($\zeta$) that captures variability between repeated oscillometry measurements, although a plurality of objective functions may be used in the method 20;
- an acceptance criterion for $\zeta$, related to oscillometry measurements.

According to an embodiment, the method may also require:

- a minimum number of oscillometry measurements to be averaged ($N_{av}$);
- a minimum number of oscillometry measurements required ($N_{min}$); and
- a maximum number of oscillometry measurements permitted ($N_{max}$).

Without loss of generality, it may be assumed that $N_{max}$ is greater than or equal to $N_{min}$, $N_{min}$ is greater than or equal to $N_{av}$, $N_{max}$ is greater than $N_{av}$, and $N_{av}$ is greater than zero. Then, the method 20 can be described as having at least some of the following steps:

At 21, upon initiation of a new test, a first oscillometry recording is taken or obtained. The recording employs an appropriate device for oscillometry measurements, as detailed in the system 10.

At 22, the oscillometry recording and/or oscillometry measurement thereof are individually validated for technical validity according to established criteria. Measurements and/or recordings that are not technically valid do not count towards $N_{min}$ and $N_{max}$.

At 23, if $N_{min}$ valid measurements have not been obtained, an additional measurement is initiated, and 21, 22 and 23 may be repeated until $N_{min}$ valid measurements are acquired. if at least $N_{min}$ and exactly $N_{av}$ valid oscillometry measurements have been obtained, $\zeta$ is(are) first evaluated for the $N_{av}$ valid oscillometry measurements at 24. if $\zeta$ meets its acceptance criterion as determined at 24 for $N_{av}$, the test is valid and complete, i.e., the acquisition of oscillometry measurements respects the standards of the test and a pulmonary function assessment may be performed based on the measurements and/or the system confirms it has successfully performed a valid oscillometry test.

According to an embodiment, the average $N_{av}$ used in the calculation of the cost function(s) $\zeta$ may be smaller to $N_{min}$, as mentioned above. As an example, $N_{av}$ is set to 3 and $N_{min}$=5. In such an example, the system 10 may take for example five measurements, resulting in possibly six permutations of three measurements (if averaging is limited to exactly $N_{av}$ measurements), or eleven permutations of three to five measurements (if averaging is allowed for any at

least $N_{av}$ measurements). The average of $N_{av}$=3 may therefore be optimized for as many as eleven possible combinations. Consequently, the results of the test may be calculated as the average of the $N_{av}$ measurements. if $\zeta$ does not meet its acceptance criterion as determined at 24, another measurement is obtained at 21.

The method 20 may include obtaining multiple oscillometry measurements in 21 before 22 and 23, such that the number of valid measurements exceeds $N_{min}$. For example, if the measurements are episodes of an oscillometry recording, the system 10 and method 20 may have numerous episodes that exceed $N_{min}$ upon obtaining the sensed data of the first oscillometry recording.

When more than $N_{min}$ valid measurements have been obtained, $\zeta$ in one embodiment is evaluated for each combination of at least $N_{av}$ measurements contained within the $>N_{av}$ available measurements, as per 25. The combinations must therefore include the averaging of at least $N_{av}$ valid measurements, but may also exceed the minimum number of $N_{av}$ valid measurements. For example, if the minimum number of $N_{av}$ valid measurements required in the average is three, and there are four valid measurements obtained (N1, N2, N3 and N4), the combinations may be as follows: (N1, N2, and N3), (N1, N3 and N4), (N2, N3 and N4), (N1, N2, N3 and N4). if $\zeta$ for at least one combination of at least $N_{av}$ valid measurements has a value that meets the acceptance criterion, the test is valid and complete, and results are calculated as the average of the at least $N_{av}$ measurements of the combination whose $\zeta$ value passes the acceptance criterion by the biggest margin. In another embodiment, $\zeta$ is evaluated for each combination of exactly $N_{av}$ measurements contained within the $>N_{av}$ available measurements at 25. In the above example, only combinations (N1, N2, and N3), (N1, N3 and N4) and (N2, N3 and N4) are evaluated; if $\zeta$ for at least one combination of exactly $N_{av}$ valid measurements has a value that meets the acceptance criterion, the test is valid and complete, and results are calculated as the average of the exactly $N_{av}$ measurements of the combination whose $\zeta$ value passes the acceptance criterion by the biggest margin.

Accordingly, step 25 may entail numerous permutations through the numerous combinations, with each permutation involving the calculation of the cost function $\zeta$. As the system 10 and method 20 must be done *in situ* to be operable, the processor(s) involved in computing the data for the system 10 and method 20 must have suitable processing speed in spite of the complexity of and volume of data computed in step 25.

As per 26, if $\zeta$ for not one combination of at least or exactly $N_{av}$ valid measurements meets the acceptance criterion, an additional recording(s) is(are) initiated, if less than the $N_{max}$ measurements have been obtained as determined at 27. Still as per 27, if $N_{max}$ measurements have been obtained and none of the $\zeta$ for any of combination of at least/exactly $N_{av}$ measurements contained within the $N_{max}$ available measurements meets the acceptance criterion as determined in 25, the test is invalid and has failed. The oscillometry test may not be used as they do not meet test standards.

The method 20 described above can function with a variety of different objective functions measured with the system 10, in the context of oscillometry measurements for subsequent pulmonary function assesment as long as at least one objection function is capturing variability between repeated oscillometry measurements. An enumeration of objective functions is provided below, and the system 10 and method 20 of the present disclosure may use one or more of the cost functions, individually, or in any appropriate combination of two or more of these cost functions. These may include:

- The coefficient of variation (CV) of the resistance at a single frequency f* that is considered representative or most important (e.g., at 5 Hz), i.e.

$$\zeta = CV(\, R(\, f^* \,) \,).$$

- The CV of the magnitude of impedance at a single frequency f* that is considered representative or most important, i.e.

$$\zeta = CV(\, |Z(\, f^* \,)| \,).$$

- The maximal CV of the resistance over a range of frequencies, i.e.

$$\zeta = \max(\, CV(\, R(\, f \,) \,) \,).$$

- The maximal CV of the magnitude of impedance over a range of frequencies, i.e.

$$\zeta = \max(\, CV(\, |Z(\, f \,)| \,) \,).$$

- An average over the values of CV(|Z|) at the $N_f$ frequencies measured, i. e.

$$\zeta = \frac{1}{N_f}\sum_{i=1}^{N_f} cv\left(\left|z_{f_i}\right|\right)$$

- Said average over the values of CV(|Z|) at the $N_f$ frequencies measured, in which an added weighing function $W$ may be used to permit attributing more importance to specific frequencies or correcting for an unevenly distributed frequency spectrum, i.e.

$$\zeta = \frac{\sum_{i=1}^{N_f} W_i \cdot CV\left(\left|Z_{f_i}\right|\right)}{\sum_{i=1}^{N_f} W_i}$$

- The Root Mean Squared (RMS) value of CV(|Z|) across the $N_f$ frequencies measured, again including an optional weighing function $W$, i.e.

$$\zeta = \left(\frac{\sum_{i=1}^{N_f} W_i \cdot CV\left(\left|Z_{f_i}\right|\right)^2}{\sum_{i=1}^{N_f} W_i}\right)^{\frac{1}{2}}$$

- A higher powered equivalent of an RMS value of CV(|Z|) across the $N_f$ frequencies measured, wherein the order of the root matches the power $p$ that each CV is elevated to, again including an optional weighing function $VV$, i.e.

$$\zeta = \left(\frac{\sum_{i=1}^{N_f} W_i \cdot CV\left(\left|Z_{f_i}\right|\right)^p}{\sum_{i=1}^{N_f} W_i}\right)^{\frac{1}{p}}$$

- The sum of the squared standard deviations divided by the sum of the squared means of |Z| across the $N_f$ frequencies measured, i.e.

$$\zeta = \frac{\sum_{i=1}^{N_f} SD\left(\left|Z_{f_i}\right|\right)^2}{\sum_{i=1}^{N_f} \left|Z_{f_i}\right|^2}$$

- Said sum of the squared standard deviations divided by the sum of the squared means of |Z| across the $N_f$ frequencies measured, with an added weighing function W, i.e.

$$\zeta = \frac{\sum_{i=1}^{N_f} W_i \cdot SD\left(\left|Z_{f_i}\right|\right)^2}{\sum_{i=1}^{N_f} W_i \cdot \left|Z_{f_i}\right|^2}$$

- The sum of the standard deviations elevated to a power $p$ divided by the sum of the means of |Z| elevated to the same power $p$, across the $N_f$ frequencies measured, again including an optional weighing function $W$, i.e.

$$\zeta = \frac{\sum_{i=1}^{N_f} W_i \cdot SD\left(\left|Z_{f_i}\right|\right)^p}{\sum_{i=1}^{N_f} W_i \cdot \left|Z_{f_i}\right|^p}$$

It should be noted that using objective functions for the CV values listed as the first four items above (with no particular order of importance), the inclusion or exclusion of a test is based on the variability at a single frequency, therefore these objective functions fail to capture the overall variability of spectral oscillometry measurements. In contrast, averaging functions as per the items after the first four items take into consideration all frequencies. The combination of a weighing factor in the average over the values of CV(|Z|) at the $N_f$ frequencies measured, and the squaring or further elevation of the summands provide mechanisms to further control the relative contributions of individual frequencies, as well as the sensitivity of the method 20 towards increased variability at only a small number of frequencies within the spectrum. Again, processing speed must support such calculations for the system 10 and method 20 to be operable *in situ*.

A commonly used acceptance criterion for inclusion or rejection of a test in the context of oscillometry measurements requires the objective function not to exceed a predefined threshold value, i.e., $\zeta \le \zeta_{max}$.

In a further embodiment, the method 20 could be extended to use a combination of a multitude of objective functions $\zeta$ related to oscillometry measurements, wherein independent acceptance criteria are applied for each objective function $\zeta$ and connected via logical operations. For example, the two objective functions

$$\zeta = \left( \frac{\sum_{i=1}^{N_f} W_i \cdot CV\left(|Z_{f_i}|\right)^P}{\sum_{i=1}^{N_f} W_i} \right)^{\frac{1}{P}}$$

and

$$\theta = \max\left( CV(|Z(f)|) \right)$$

could be meaningfully combined so that tests are accepted when $\zeta \le \zeta_{max}$ and $\theta \le \Theta_{max}$.

In another embodiment, the method 20 may employ quality grading to determine if the oscillometry measurements are suitable for a proper assessment of pulmonary function. The method 20 may employ quality grading by introducing multiple thresholds $\zeta_j$, so that $\zeta_1 < \zeta_2 < ... < \zeta_{max}$, wherein passing the most stringent threshold $\zeta_1$ results in the best possible rating. For the example of a total of three thresholds (including $\zeta_{max}$), this would result in the following grading:

| Value of $\zeta$ | Rating |
|---|---|
| $\le \zeta_1$ | "A" - Excellent |
| $> \zeta_1$ but $\le \zeta_2$ | "B" - Good |
| $> \zeta_2$ but $\le \zeta_{max}$ | "C" - Fair |
| $> \zeta_{max}$ | "X" - Rejected |

In another embodiment, thresholds on $\zeta$ are combined with other factors, such as the number of measurements required to reach a certain grading. For the example of $N_{min} = 3$, $N_{max} = 5$ and a total of three thresholds (including $\zeta_{max}$), this would result in the following grading:

| | | Number of Measurements Needed | | |
|---|---|---|---|---|
| | | 3 | 4 | 5 |
| Value of $\zeta$ | $\le \zeta_1$ | "A" – Excellent | "A" – Excellent | "B" – Good |
| | $> \zeta_1$ but $\le \zeta_2$ | "B" – Good | "B" – Good | "C" – Fair |
| | $> \zeta_2$ but $\le \zeta_{max}$ | "C" – Fair | "C" – Fair | "C" – Fair |
| | $> \zeta_{max}$ | "X" – Rejected | "X" – Rejected | "X" – Rejected |

In another embodiment where multiple objective functions are used, multiple thresholds per objective functions can be combined to produce a grading. For the example of a total of three thresholds for two objective functions $\zeta$ and $\Theta$, this would result in the following grading:

| | | Value of $\Theta$ | | | |
|---|---|---|---|---|---|
| | | $\leq \Theta_1$ | $> \Theta_1$ but $\leq \Theta_2$ | $> \Theta_2$ but $\leq \Theta_{max}$ | $> \Theta_{max}$ |
| Value of $\zeta$ | $\leq \zeta_1$ | "A" – Excellent | "A" – Excellent | "B" – Good | "X" – Rejected |
| | $> \zeta_1$ but $\leq \zeta_2$ | "A" – Excellent | "B" – Good | "C" – Fair | "X" – Rejected |
| | $> \zeta_2$ but $\leq \zeta_{max}$ | "B" – Good | "C" – Fair | "X" – Rejected | "X" – Rejected |
| | $> \zeta_{max}$ | "X" – Rejected | "X" – Rejected | "X" – Rejected | "X" – Rejected |

In another embodiment, the grading scheme is calculated as a decimal score S that assumes its maximal value $S_{max}$ when variability is zero and approaches zero as $\zeta$ approaches $\zeta_{max,}$ i.e.

$$S = \begin{cases} S_{max} \cdot \dfrac{\zeta_{max} - \zeta}{\zeta_{max}} & \text{for } \zeta \leq \zeta_{max} \\ 0 & \text{for } \zeta > \zeta_{max} \end{cases}$$

Accordingly, the methods of the present disclosure may provide, in addition or as an alternative to a binary pass/fail value, a grading of the oscillometry measurements. In particular, the methods may include the use of one or more objective functions, to determine if the oscillometry measurements are suitable for a proper assessment of pulmonary function. Likewise, the system 10 of the present disclosure has the capacity of performing a self-assessment of oscillometry measurements performed thereon - with suitable processing speed -, to assist an operator in the decision making regarding the quality of the pulmonary function tests on a subject.

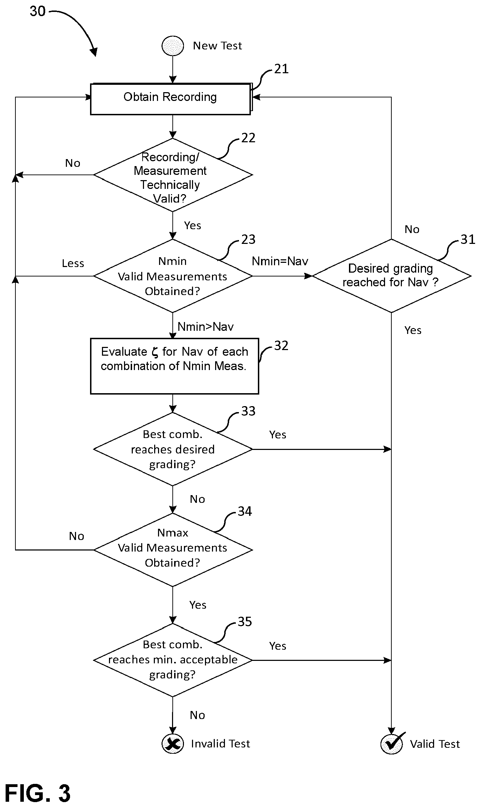

In the presence of a grading scheme as described above, the method 20 described above and shown in Fig. 2 may be extended to include two thresholds, in the manner shown as 30 in Fig. 3, still in the context of oscillometry measurements. As methods 20 and 30 share some steps, like reference numerals will indicate similar steps. The two thresholds of method 30 may be:

- a more stringent "desired grading" that the method aims to reach, and

- a less stringent "minimum acceptable grading" that must be reached for a test to be considered valid.

With reference to Fig. 3, a patient test according to the method 30 may proceed as follows. In similar fashion to method 20, at 21, upon initiation of a new test, a first oscillometry recording and/or measurement is taken. At 22, the measurement is individually validated for technical validity according to established criteria. Measurements that are not technically valid do not count towards $N_{min}$ and $N_{max}$. At 23, when $N_{min}$ valid measurements have been obtained in a condition of $N_{av} = N_{min}$, $\zeta$ is(are) first evaluated and graded at 31, such that a preliminary grading is established. Still in 30, if the preliminary grading reaches or exceeds the desired grading upon comparison with the threshold(s), the test is valid and complete, and results are calculated as the average of the $N_{av}$ measurements. The oscillometry measurements are suitable for a proper assessment of pulmonary function, and the acquisition by the method 30 is completed, for the assessment of pulmonary mechanics to be performed based on the oscillometry measurements. if $\zeta$ does not meet the desired grading as determined at 30, another measurement is obtained at 21.

The method 30 may include obtaining multiple measurements in 21 before 22 and 23, such that the number of valid measurements exceeds $N_{min}$. As another possibility, $N_{av} < N_{min}$ as detailed above in steps 23 and 25. When more than $N_{min}$ valid measurements and/or more than $N_{av}$ valid measurements have been obtained, the objective function $\zeta$ in 32 is evaluated and a preliminary grading is attributed to the average of each combination of at least $N_{av}$ measurements (or exactly $N_{av}$ measurements) contained within the available measurements, as detailed above for 25. In 33, if the grading for $N_{av}$ of at least one combination meets at least the desired grading, the test is valid and complete, and results are calculated as the average of the $N_{av}$ measurements of the combination having at least $N_{av}$ measurements (or exactly $N_{av}$ measurements) with the highest grading (if the grading is quantitative), or that passes the desired grading threshold by the biggest margin (if the grading is binned, e.g. for a letter grading).

In 34, if the average of not one combination of at least $N_{av}$ valid measurements meets the desired grading, one or more additional recordings are initiated at 21, if less than $N_{max}$ measurements have been obtained, as per 34. The additional measurement(s) may serve to improve the grading of the oscillometry measurement. Still in 34, if $N_{max}$ measurements have been obtained and the average of none of the combinations of at least $N_{av}$ measurements (or exactly $N_{av}$ measurements) contained within the various $N_{max}$ available measurements reaches the desired grading, the method 30 reaches 35. According to 35, if at least one grading reaches or exceeds a minimum acceptable grading, the test is valid and complete, and results are calculated as the average of those at least $N_{av}$ measurements (or exactly $N_{av}$ measurements) with the highest grading (if the grading is quantitative), or that passes the minimum acceptable grading threshold by the biggest margin (if the grading is binned, e.g. for a letter grading). Still at 35, if the minimum acceptable grading threshold is not reached by any combination of measurements, the test is invalid and has failed.

As described above, the methods 20 and 30 may apply to oscillometry recordings as a whole, or to isolated episodes. In the latter case, with reference to Fig. 4, the system 10 may or may not apply a method 40 for isolating some episodes within an oscillometry recording, to perform the assessment on isolated episodes such as breaths rather than entire oscillometry recordings. As methods 20, 30 and 40 share some steps, like reference numerals will indicate similar steps. More specifically, the method 20 described above and shown in Fig. 2 may be extended to include the additional step 41 of isolating such episodes. The isolating of episodes may for example be performed by a review of the data of oscillometry recordings to identify markers delimiting an episode. For example, the markers may include maxima, minima, zero crossings or crossings of pre-defined threshold values of the flow or volume signals, to name a few of many possible options. Processing speed must support such isolation for the system 10 and method 40 to be operable *in situ.* Although not shown, the step 41 could be added to the method 30 of Fig. 3 as well, with the step 41 occurring for example between steps 21 and 22.

As described above, the device A may be controlled in such a way that the onset of the oscillometry recording (i) always coincides with the same point in the breathing cycle, and/or (ii) occurs only if conditions to confirm stability of the breathing pattern have been met. Therefore, the method 20 described above and shown in Fig. 2 may be extended into method 50 of Fig. 5. The method 50 includes the additional step 51 of monitoring the breathing pattern until the desired point in the breathing cycle is reached. The step 51 may trigger the step 21 of obtaining the oscillometry recording, for instance synchronized with a predetermined episode, by monitoring any one of the markers identified above for 41, or by predictive timing based on previous episodes. For example, if the system determines that a given patient has a stable breath duration of six seconds, a recording can be triggered five seconds into a breath to be certain that a new breath starts early in the recording. Processing speed must support such monitoring and triggering for the system 10 and method 50 to be operable *in situ.* Although not shown, the step 51 could be added to the method 30 of Fig. 3 as well, with the step 51 occurring before step 21.

The methods 20, 30, 40 and 50 of Figs. 2, 3, 4 and 5, respectively, may be integrated into the processor B of the system 10. The processor B or processors B of the system 10 may include a non-transitory computer-readable memory communicatively coupled to the processing unit(s) B and comprising computer-readable program instructions executable by the processing unit for executing at least some of the steps of methods 20, 30, 40 and 50. The various steps of the methods 20, 30, 40 and 50 may be executed by different modules of the system 10. At the outset, the system 10 therefore aims to output oscillometry measurements that comply with an established testing protocol and standards in situ. This allows corrective measures and a suitable number of oscillometry measurements to be taken during a clinical session, to avoid having to retest a patient at a later time.

In the methods 20, 30, 40 and 50 described above, the concepts of minimum number $N_{min}$ of oscillometry measurements and average $N_{av}$ of a number of oscillometry measurements are applied. In all of these methods, the average $N_{av}$ used in the calculation of the cost function(s) may be smaller or equal to $N_{min}$, in addition to having the capacity of being higher as well. As an example, $N_{av}$ is set to n=3 and $N_{min}$=5, for any of methods 20, 30, 40 and 50. In such an example, the system 10 may take for example five measurements, resulting in possibly six permutations of three measurements (if averaging is limited to exactly $N_{av}$ measurements), or eleven permutations of three to five measurements (if averaging is allowed for any at least $N_{av}$ measurements). The average of $N_{av}$=3 may therefore be optimized for as many as eleven possible combinations.

As another possibility, $N_{av}$ and $N_{min}$ are equal to one another, as explored with steps 24 (Fig. 2) and 31 (Fig. 3). In the example, they are both equal to three, but the system 10 may take more than three measurements in a >$N_{min}$ scenario (e.g., five measurements), whereby $N_{av}$ may be optimized when selecting among the eleven combinations. Consequently, in such an embodiment, some degree of optimization is possible if more than $N_{min}$ measurement are taken.

EXEMPLARY TESTING DATA

For illustrative purposes only, to illustrate the system 10 and some aspects of the methods described herein, exemplary testing data is provided below. As the testing data was obtained in particular settings, it should only be viewed as a particular non-limitative embodiment.

To test method 20, from a larger dataset, 40 high-variability patients (including a variety of pathologies) were extracted, for whom tests containing at least four oscillometry measurements had been recorded. First, a calculation

EP 3 658 021 B1

was performed for both the CV of impedance magnitude at a measurement frequency of 5 Hz, $CV(Z_5)$, and the Root Mean Squared value of $CV(|Z|)$ across all measured frequencies, $CV_{rms}(Z)$, as described above (and, in case of the $CV_{rms}(Z)$, with all weights equalling 1.0). $CV(Z_5)$ and $CV_{rms}(Z)$ were each evaluated both across all measurements collected, representing the unqualified tests results as might be produced by an unskilled or novice user.

Next, each test was examined by an expert physician and researcher with ample experience in reviewing oscillometry data, with no time constraint as such review would far exceed any *in situ* assessment and would not be possible in the conditions of operation of the system 10 and methods 20, 30, 40 and 50. For 35 of the patient tests, the expert manually selected three valid measurements, and $CV(Z_5)$ and $CV_{rms}(Z)$ were each evaluated across the selected measurements, representing the qualified test results produced by an expert user.

Finally, the method 20 was applied to 34 out of the 35 patient tests selected by the expert, using $N_{av} = 3$, $N_{min} = 5$ and $N_{max} = 5$. One test was excluded from further analysis because it contained only 4 measurements and therefore did not comply with $N_{min} = 5$. $CV(Z_5)$ and $CV_{rms}(Z)$ were each evaluated across the measurements selected by method 20, representing the automatically qualified test results produced by our method.

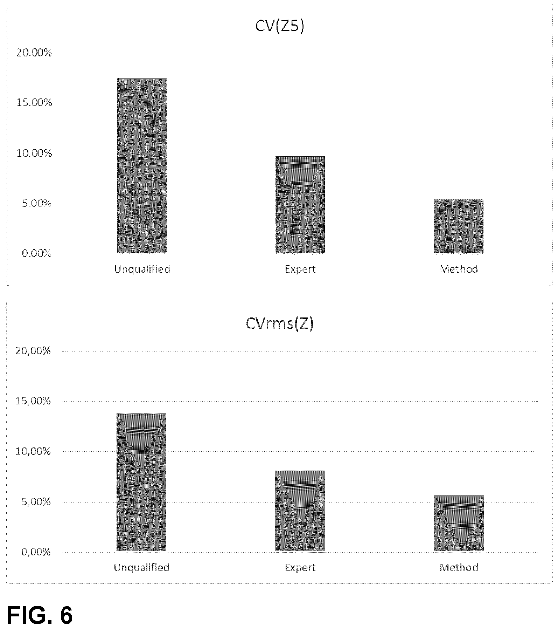

Fig. 6 shows the average CVs for all three cases. A substantial and significant (paired t-test; all $p<0.00001$) difference can be observed between the unqualified and the expert-qualified test results for both $CV(Z_5)$ and $CV_{rms}(Z)$ that underscores the importance of a good qualification method on the reproducibility of outcomes and illustrates the dependence of the prior art on operator skill, in non *in situ* settings as it is not possible to perform such human analysis in the conditions set out above for the system 10 and methods 20, 30, 40 and 50.

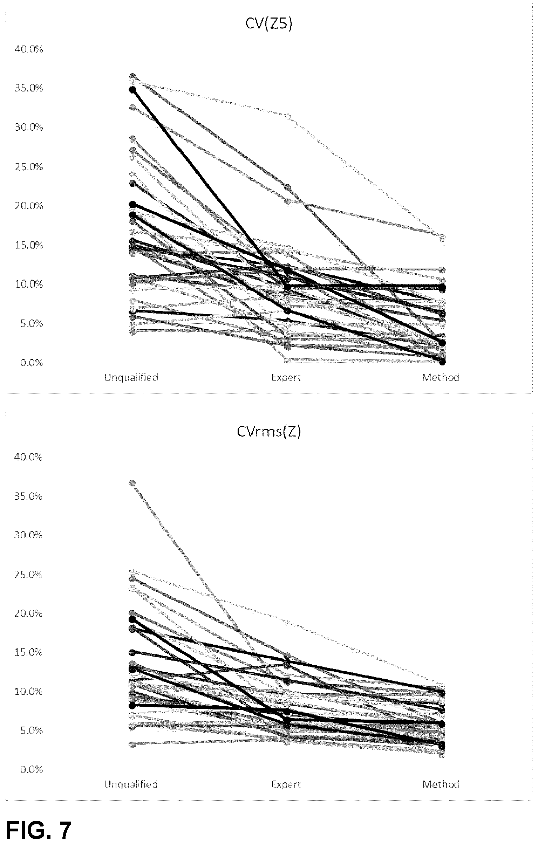

Fig. 6 also shows that the automated qualification using method 20 produced coefficients of variation that were lower (all $p<0.00001$) than both the corresponding expert-qualified values and the unqualified values, for both $CV(Z_5)$ and $CV_{rms}(Z)$, in addition to being performable *in situ,* e.g., in real-time or quasi-real time. On average, as an example only, the method 20 improved $CV(Z_5)$ and $CV_{rms}(Z)$ by 12.1% and 8.1%, respectively, whereas the expert improved $CV(Z_5)$ and $CV_{rms}(Z)$ by a lesser 7,8% and 5.7%, respectively. As illustrated in Fig. 7, method 20 improved both $CV(Z_5)$ and $CV_{rms}(Z)$ for every one of the 34 patient tests, whereas the expert failed to improve or even worsened $CV(Z_5)$ and $CV_{rms}(Z)$ in 5 and 4 cases, respectively.

To establish exclusion thresholds, statistics were performed on the expert-qualified data to identify the values that correspond to the 95th percentile, which for $CV(Z_5)$ and $CV_{rms}(Z)$ equalled 15.2% and 15.8%, respectively. Applying the threshold for $CV(Z_5)$ to the unqualified, expert-qualified and method 20-qualified datasets resulted in 17, 3 and 2 excluded tests, respectively, whereas applying the threshold for $CV_{rms}(Z)$ to the three datasets yielded 10, 1 and zero excluded tests, respectively.

To further test the method 30 in the same exemplary dataset, thresholds were also established corresponding to the 75th and 85th percentile of the expert-qualified data, so that patient test could be graded as follows:

| Expert-qualified percentile | $CV(Z_5)$ | $CV_{rms}(Z)$ | Grading |
|---|---|---|---|
| 75% | < 11.2% | < 9.7% | A |
| 85% | < 12.6% | < 11.7% | B |
| 95% | < 15.2% | < 15.8% | C |
| | ≥ 15.2% | ≥ 15.8% | X |

Applying the thresholds for $CV(Z_5)$, the grading was as follows:

| Grading | Unqualified | Expert | Method 30 |
|---|---|---|---|
| A | 11 | 23 | 31 |
| B | 0 | 5 | 1 |
| C | 6 | 3 | 0 |
| X | 17 | 3 | 2 |

Applying the thresholds for $CV_{rms}(Z)$, the grading was as follows:

| Grading | Unqualified | Expert | Method 30 |
|---|---|---|---|
| A | 10 | 25 | 30 |

(continued)

| Grading | Unqualified | Expert | Method 30 |
|---------|-------------|--------|-----------|
| B | 8 | 4 | 4 |
| C | 6 | 4 | 0 |
| X | 10 | 1 | 0 |

The data presented above illustrate that methods 20, 30, 40 and 50 offer means for automatic in-situ quality control and test workflow management that cannot be perform by a human operator due to the demand of analytical evaluations and decisions..

EXEMPLARY OSCILLOMETRY MEASUREMENT DEVICE A

The oscillometry measurement device A of Fig. 1 may be embodied for instance in the manner shown in Fig. 8. However, this is merely an option among others for obtaining oscillometry measurements with the system 10 or methods 20, 30, 40 and 50 of the present disclosure. Other exemplary devices are described above.

With the oscillometry measurement device A, a subject breathes through the device A via a mouthpiece 80 connected to a duct 81 via a bacterial filter 82. The other side of duct 81 is connected to an oscillator 90 that may include an oscillator housing 91, an oscillator piston 92, a linear actuator 93 capable of oscillating piston 92, and an atmosphere port 94 with a defined impedance to atmosphere. The interior of the oscillator 90 contains a front chamber 95 that communicates with the airway opening and is subject to the pressure swings generated by the oscillator 90 and the subject's breathing. The front chamber 95 further contains a sensor 96 for measuring flow and a sensor 97 for measuring pressure. Analog/digital converters and digital/analog converters 98 interface sensors 96 and 97 as well as a power amplifier 99 driving the actuator 44 to a processor 100. The processor 100 may or may not be part of the processor B. In an embodiment, the processor 100 is a microprocessor integrated to the device A such that the device A may be separate from the processor B, with the processor B receiving signals from the processor 100.

## Claims

1. A method (20, 30, 40, 50) for acquiring oscillometry measurements with an oscillometry measuring system (10) in lung function assessment, the method comprising:

   receiving (21) repeated oscillometry measurements, using at least one processor (100) of the oscillometry measuring system (10), the repeated oscillometry measurements being from at least one oscillometry recording;
   identifying (21), using the at least one processor (100) of the oscillometry measuring system (10), parameters in the repeated oscillometry measurements;
   calculating (25), using the at least one processor (100) of the oscillometry measuring system (10), at least one objective function from the parameters of the repeated oscillometry measurements, the at least one objective function capturing variability between the repeated oscillometry measurements,
   evaluating (25), using the at least one processor of the oscillometry measuring system (10), the at least one objective function as a function of at least one predetermined threshold;
   based on the results of the evaluating (25), accepting or rejecting (26), using the at least one processor (100) of the oscillometry measuring system (10), the repeated oscillometry measurements; and
   outputting, using the at least one processor (100) of the oscillometry measuring system (10), oscillometry data using the oscillometry measurements if accepted from the evaluating (25).

2. The method (20, 30, 40, 50) according to claim 1, wherein receiving (21) repeated oscillometry measurements includes receiving repeated oscillometry measurements delimited by breathing episodes.

3. The method (20, 30, 40, 50) according to claim 2, wherein receiving (21) oscillometry measurements delimited by breathing episodes includes isolating the breathing episodes from the at least one oscillometry recording and wherein optionally, isolating the breathing episodes includes identifying a marker in the at least one oscillometry recording including at least one of maxima, minima, zero crossings, crossings of pre-defined threshold values of flow or volume signals.

4. The method (20, 30, 40, 50) according to claim 2, wherein receiving (21) oscillometry measurements delimited by breathing episodes includes monitoring a breath of a subject and triggering a recording of the oscillometry measurements upon detection of a desired point in a breathing cycle and wherein, optionally, triggering a recording of the oscillometry measurements includes identifying a marker in the monitoring including at least one of maxima, minima, zero crossings, crossings of pre-defined threshold values of flow or volume signals.

5. The method (20, 30, 40, 50) according to any one of claims 1 to 4, wherein (21) receiving repeated oscillometry measurements includes recording the oscillometry measurements during the at least one oscillometry recording using an oscillometry measurement device from the oscillometry measuring system.

6. The method (20, 30, 40, 50) according to any one of claims 1 to 5, wherein identifying the parameters in the repeated oscillometry measurements includes identifying at least one of respiratory system resistance, reactance or impedance for the repeated oscillometry measurements.

7. The method (20, 30, 40, 50) according to any one of claims 1 to 6, wherein calculating (25) at least one objective function includes calculating a coefficient of variation (CV) of resistance at a single frequency $f^*$ according to $\zeta = CV(R(f^*))$.

8. The method (20, 30, 40, 50) according to any one of claims 1 to 7, wherein calculating (25) at least one objective function includes calculating a coefficient of variation (CV) of impedance at a single frequency $f^*$ according to $\zeta = CV(|Z(f^*)|)$.

9. The method (20, 30, 40, 50) according to any one of claims 1 to 8, wherein calculating (25) at least one objective function includes calculating a maximum coefficient of variation (CV) of a resistance over a range of frequencies according to $\zeta = \max(CV(R(f)))$.

10. The method (20, 30, 40, 50) according to any one of claims 1 to 9, wherein calculating (25) at least one objective function includes calculating a maximum coefficient of variation (CV) of an impedance over a range of frequencies according to $\zeta = \max(CV(|Z(f)|))$.

11. The method (20, 30, 40, 50) according to any one of claims 1 to 10, wherein calculating (25) at least one objective function includes calculating an average of coefficients of variation (CV) of an impedance over $N_f$ frequencies measured according to

$$\zeta = \frac{1}{N_f}\sum_{i=1}^{N_f} CV\left(\left|z_{f_i}\right|\right)$$

and wherein, optionally, calculating at least one objective function includes calculating said average of coefficients of variation (CV) of the impedance over $N_f$ frequencies by adding a weighing function W according to

$$\zeta = \frac{\sum_{i=1}^{N_f} W_i \cdot CV\left(\left|Z_{f_i}\right|\right)}{\sum_{i=1}^{N_f} W_i}.$$

12. The method (20, 30, 40, 50) according to claim 11, wherein calculating (25) at least one objective function includes calculating said average of coefficients of variation (CV) of the impedance over $N_f$ frequencies by calculating a root mean squared value according to

$$\zeta = \left(\frac{\sum_{i=1}^{N_f} W_i \cdot CV\left(\left|Z_{f_i}\right|\right)^2}{\sum_{i=1}^{N_f} W_i}\right)^{\frac{1}{2}}.$$

and/or, wherein calculating at least one objective function includes calculating said average of coefficients of variation (CV) of the impedance over $N_f$ frequencies by calculating a root mean squared value in which an order of the root marches a power p to which each CV is elevated to according to

$$\zeta = \left( \frac{\sum_{i=1}^{N_f} W_i \cdot CV(|Z_{f_i}|)^{\mathrm{p}}}{\sum_{i=1}^{N_f} W_i} \right)^{\frac{1}{p}}.$$

13. The method (20, 30, 40, 50) according to any one of claims 1 to 12, wherein calculating (25) at least one objective function includes calculating a sum of squared standard deviations divided by a sum of squared means of |Z| across $N_f$ frequencies measured according to

$$\zeta = \frac{\sum_{i=1}^{N_f} SD(|Z_{f_i}|)^2}{\sum_{i=1}^{N_f} \overline{|Z_{f_1}|}^2}.$$

14. The method (20, 30, 40, 50) according to any one of claims 1 to 13, wherein calculating (25) at least one objective function includes calculating a sum of squared standard deviations divided by a sum of squared means of |Z| across $N_f$ frequencies measured, with an added weighing function $W$ according to

$$\zeta = \frac{\sum_{i=1}^{N_f} W_i \cdot SD(|Z_{f_i}|)^2}{\sum_{i=1}^{N_f} W_i \cdot \overline{|Z_{f_i}|}^2}.$$

and/or, wherein calculating at least one objective function includes calculating a sum of standard deviations elevated to a power $p$ divided by a sum of means of |Z| elevated to the power $p$, across $N_f$ frequencies measured, including a weighing function $W$ according to

$$\zeta = \frac{\sum_{i=1}^{N_f} W_i \cdot SD(|Z_{f_i}|)^{\mathrm{p}}}{\sum_{i=1}^{N_f} W_i \cdot \overline{|Z_{f_i}|}^{\mathrm{p}}}.$$

15. The method (20, 30, 40, 50) according to any one of claims 1 to 14, wherein calculating (25) at least one objective function includes determining (23) if a minimum number $N_{min}$ of oscillometry measurements is reached prior to calculating the at least one objective function.

16. The method (20, 30, 40, 50) according to claim 15, further comprising combining oscillometry measurements if the minimum number $N_{min}$ of oscillometry measurements is exceeded, the combining include all permutations having at least a minimum number $N_{av}$ of oscillometry measurements required for averaging.

17. The method (20, 30, 40, 50) according to claim 16, wherein evaluating (25) the at least one objective function includes evaluating the at least one objective function using one of the permutations selected as a function of the at least one predetermined threshold.

18. The method (20, 30, 40, 50) according to any one of claims 15 to 17, wherein calculating (25) at least one objective function includes calculating the objective function using an average of the parameters for the oscillometry measurements.

19. A system (10) for acquiring oscillometry measurements in lung function assessment, the system comprising:

a processing unit (100); and
a non-transitory computer-readable memory communicatively coupled to the processing unit and comprising computer-readable program instructions executable by the processing unit (100) for:

receiving (21) repeated oscillometry measurements, using at least one processor (100) of the oscillometry measuring system (10), the repeated oscillometry measurements being from at least one oscillometry recording;

identifying (21), using the at least one processor (100) of the oscillometry measuring system (10), parameters in the repeated oscillometry measurements;

calculating (25), using the at least one processor (100) of the oscillometry measuring system (10), at least one objective function from the parameters of the repeated oscillometry measurements, the at least one objective function capturing variability between the repeated oscillometry measurements,

evaluating (25), using the at least one processor (100) of the oscillometry measuring system (10), the at least one objective function as a function of at least one predetermined threshold;

based on the results of the evaluating (25), accepting or rejecting (26), using the at least one processor (100) of the oscillometry measuring system (10), the repeated oscillometry measurements; and

outputting (C), using the at least one processor (100) of the oscillometry measuring system (10), oscillometry data using the oscillometry measurements if accepted from the evaluating (25).

## Patentansprüche

1.  Verfahren (20, 30, 40, 50) zum Erfassen von Oszillometrie-Messwerten mit einem Oszillometrie-Messsystem (10) bei der Lungenfunktionsbeurteilung, wobei das Verfahren Folgendes beinhaltet:

    Empfangen (21) von wiederholten Oszillometrie-Messwerten durch mindestens einen Prozessor (100) des Oszillometrie-Messsystems (10), wobei die wiederholten Oszillometrie-Messwerte von mindestens einer Oszillometrie-Aufzeichnung stammen;
    Identifizieren (21), durch den mindestens einen Prozessor (100) des Oszillometrie-Messsystems (10), von Parametern in den wiederholten Oszillometrie-Messwerten;
    Berechnen (25), durch den mindestens einen Prozessor (100) des Oszillometrie-Messsystems (10), mindestens einer Zielfunktion aus den Parametern der wiederholten Oszillometrie-Messwerte, wobei die mindestens eine Zielfunktion Variabilität zwischen den wiederholten Oszillometrie-Messwerten erfasst,
    Beurteilen (25), durch den mindestens einen Prozessor des Oszillometrie-Messsystems (10), der mindestens einen Zielfunktion in Abhängigkeit von mindestens einem vorbestimmten Schwellenwert;
    Akzeptieren oder Zurückweisen (26) der wiederholten Oszillometrie-Messwerte auf der Basis der Ergebnisse des Beurteilens (25) durch den mindestens einen Prozessor (100) des Oszillometrie-Messsystems (10); und
    Ausgeben, durch den mindestens einen Prozessor (100) des Oszillometrie-Messsystems (10), von Oszillometrie-Daten anhand der Oszillometrie-Messwerte, wenn diese von dem Beurteilen (25) akzeptiert werden.

2.  Verfahren (20, 30, 40, 50) nach Anspruch 1, wobei das Empfangen (21) von wiederholten Oszillometrie-Messwerten das Empfangen von durch Atmungsepisoden begrenzten wiederholten Oszillometrie-Messwerten beinhaltet.

3.  Verfahren (20, 30, 40, 50) nach Anspruch 2, wobei das Empfangen (21) von durch Atmungsepisoden begrenzten Oszillometrie-Messwerten das Isolieren der Atmungsepisoden aus der mindestens einen Oszillometrie-Aufzeichnung beinhaltet, und wobei das Isolieren der Atmungsepisoden optional das Identifizieren einer Markierung in der mindestens einen Oszillometrie-Aufzeichnung beinhaltet, einschließlich mindestens eines von Maxima, Minima, Nulldurchgängen, Durchgängen von vordefinierten Schwellenwerten von Durchfluss- oder Volumensignalen.

4.  Verfahren (20, 30, 40, 50) nach Anspruch 2, wobei das Empfangen (21) von durch Atmungsepisoden begrenzten Oszillometrie-Messwerten das Überwachen eines Atems eines Subjekts und das Auslösen einer Aufzeichnung der Oszillometrie-Messwerte nach dem Erkennen eines gewünschten Punkts in einem Atmungszyklus beinhaltet, und wobei das Auslösen einer Aufzeichnung der Oszillometrie-Messwerte optional das Identifizieren einer Markierung in der Überwachung beinhaltet, einschließlich mindestens eines von Maxima, Minima, Nulldurchgängen, Durchgängen von vordefinierten Schwellenwerten von Durchfluss- oder Volumensignalen.

5.  Verfahren (20, 30, 40, 50) nach einem der Ansprüche 1 bis 4, wobei das Empfangen (21) von wiederholten Oszillometrie-Messwerten das Aufzeichnen der Oszillometrie-Messwerte während der mindestens einen Oszillometrie-Aufzeichnung durch ein Oszillometrie-Messgerät von dem Oszillometrie-Messsystem beinhaltet.

6.  Verfahren (20, 30, 40, 50) nach einem der Ansprüche 1 bis 5, wobei das Identifizieren der Parameter in den wiederholten Oszillometrie-Messwerten das Identifizieren von mindestens einem von Atemwegswiderstand, Reaktanz oder Impedanz für die wiederholten Oszillometrie-Messwerte beinhaltet.

7. Verfahren (20, 30, 40, 50) nach einem der Ansprüche 1 bis 6, wobei das Berechnen (25) mindestens einer Zielfunktion das Berechnen eines Variationskoeffizienten (CV) von Widerstand bei einer einzelnen Frequenz $f^*$ gemäß $\zeta = CV(R(f^*))$ beinhaltet.

8. Verfahren (20, 30, 40, 50) nach einem der Ansprüche 1 bis 7, wobei das Berechnen (25) mindestens einer Zielfunktion das Berechnen eines Variationskoeffizienten (CV) von Impedanz bei einer einzelnen Frequenz $f^*$ gemäß $\zeta = CV(|Z(f^*)|)$ beinhaltet.

9. Verfahren (20, 30, 40, 50) nach einem der Ansprüche 1 bis 8, wobei das Berechnen (25) mindestens einer Zielfunktion das Berechnen eines maximalen Variationskoeffizienten (CV) eines Widerstands über einen Frequenzbereich gemäß $\zeta = \max(CV(R(f)))$ beinhaltet.

10. Verfahren (20, 30, 40, 50) nach einem der Ansprüche 1 bis 9, wobei das Berechnen (25) mindestens einer Zielfunktion das Berechnen eines maximalen Variationskoeffizienten (CV) einer Impedanz über einen Frequenzbereich gemäß $\zeta = \max(CV(|Z(f)|))$ beinhaltet.

11. Verfahren (20, 30, 40, 50) nach einem der Ansprüche 1 bis 10, wobei das Berechnen (25) mindestens einer Zielfunktion das Berechnen eines Durchschnitts von Variationskoeffizienten (CV) einer Impedanz über $N_f$ gemessene Frequenzen beinhaltet, gemäß

$$\zeta = \frac{1}{N_f}\sum_{i=1}^{N_f} CV(|z_{f_i}|)$$

und wobei das Berechnen mindestens einer Zielfunktion optional das Berechnen des genannten Durchschnitts von Variationskoeffizienten (CV) der Impedanz über $N_f$ Frequenzen durch Addieren einer Gewichtungsfunktion $W$ gemäß

$$\zeta = \frac{\sum_{i=1}^{N_f} W_i \cdot CV(|Z_{f_i}|)}{\sum_{i=1}^{N_f} W_i}$$

beinhaltet.

12. Verfahren (20, 30, 40, 50) nach Anspruch 11, wobei das Berechnen (25) mindestens einer Zielfunktion das Berechnen des genannten Durchschnitts von Variationskoeffizienten (CV) der Impedanz über $N_f$ Frequenzen durch Berechnen eines Effektivwerts gemäß

$$\zeta = \left(\frac{\sum_{i=1}^{N_f} W_i \cdot CV(|z_{f_i}|)^2}{\sum_{i=1}^{N_f} W_i}\right)^{\frac{1}{2}}$$

beinhaltet, und/oder wobei das Berechnen mindestens einer Zielfunktion das Berechnen des genannten Durchschnitts von Variationskoeffizienten (CV) der Impedanz über $N_f$ Frequenzen durch Berechnen eines Effektivwerts beinhaltet, wobei eine Ordnung der Wurzel einer Potenz p entspricht, zu der jeder CV erhoben ist, gemäß

$$\zeta = \left(\frac{\sum_{i=1}^{N_f} W_i \cdot CV(|z_{f_i}|)^p}{\sum_{i=1}^{N_f} W_i}\right)^{\frac{1}{p}}.$$

**13.** Verfahren (20, 30, 40, 50) nach einem der Ansprüche 1 bis 12, wobei das Berechnen (25) mindestens einer Zielfunktion das Berechnen einer Summe von quadrierten Standardabweichungen dividiert durch eine Summe von quadrierten Mittelwerten von |Z| über $N_f$ gemessene Frequenzen beinhaltet, gemäß

$$\zeta = \frac{\sum_{i=1}^{N_f} SD\left(\left|Z_{f_i}\right|\right)^2}{\sum_{i=1}^{N_f} \overline{\left|Z_{f_i}\right|}^2} .$$

**14.** Verfahren (20, 30, 40, 50) nach einem der Ansprüche 1 bis 13, wobei das Berechnen (25) mindestens einer Zielfunktion das Berechnen einer Summe von quadrierten Standardabweichungen dividiert durch eine Summe von quadrierten Mittelwerten von |Z| über $N_f$ gemessene Frequenzen beinhaltet, mit einer zusätzlichen Gewichtungsfunktion W gemäß

$$\zeta = \frac{\sum_{i=1}^{N_f} W_i \cdot SD\left(\left|Z_{f_i}\right|\right)^2}{\sum_{i=1}^{N_f} W_i \cdot \overline{\left|Z_{f_i}\right|}^2} .$$

und/oder wobei das Berechnen mindestens einer Zielfunktion das Berechnen einer Summe von zu einer Potenz p erhobenen Standardabweichungen dividiert durch eine Summe von zur Potenz p erhobenen Mittelwerten von |Z| über $N_f$ gemessene Frequenzen beinhaltet, einschließlich einer Gewichtungsfunktion $W$ gemäß

$$\zeta = \frac{\sum_{i=1}^{N_f} W_i \cdot SD\left(\left|Z_{f_i}\right|\right)^p}{\sum_{i=1}^{N_f} W_i \cdot \overline{\left|Z_{f_i}\right|}^p} .$$

**15.** Verfahren (20, 30, 40, 50) nach einem der Ansprüche 1 bis 14, wobei das Berechnen (25) mindestens einer Zielfunktion das Feststellen (23) beinhaltet, ob eine Mindestanzahl $N_{min}$ von Oszillometrie-Messwerten vor dem Berechnen der mindestens einen Zielfunktion erreicht ist.

**16.** Verfahren (20, 30, 40, 50) nach Anspruch 15, das ferner das Kombinieren von Oszillometrie-Messwerten beinhaltet, wenn die Mindestanzahl $N_{min}$ von Oszillometrie-Messwerten überschritten wird, wobei das Kombinieren alle Permutationen mit mindestens einer Mindestanzahl $N_{av}$ von Oszillometrie-Messwerten umfasst, die für die Durchschnittsbildung erforderlich ist.

**17.** Verfahren (20, 30, 40, 50) nach Anspruch 16, wobei das Beurteilen (25) der mindestens einen Zielfunktion das Beurteilen der mindestens einen Zielfunktion anhand einer der in Abhängigkeit von dem mindestens einen vorbestimmten Schwellenwert ausgewählten Permutationen beinhaltet.

**18.** Verfahren (20, 30, 40, 50) nach einem der Ansprüche 15 bis 17, wobei das Berechnen (25) mindestens einer Zielfunktion das Berechnen der Zielfunktion anhand eines Durchschnitts der Parameter für die Oszillometrie-Messwerte beinhaltet.

**19.** System (10) zum Erfassen von Oszillometrie-Messwerten bei der Lungenfunktionsbeurteilung, wobei das System Folgendes umfasst:

    eine Verarbeitungseinheit (100); und
    einen nichtflüchtigen, computerlesbaren Speicher, der kommunikativ mit der Verarbeitungseinheit gekoppelt ist und computerlesbare Programmbefehle enthält, die von der Verarbeitungseinheit (100) ausgeführt werden können zum:

Empfangen (21) von wiederholten Oszillometrie-Messwerten durch mindestens einen Prozessor (100) des Oszillometrie-Messsystems (10), wobei die wiederholten Oszillometrie-Messwerte aus mindestens einer Oszillometrie-Aufzeichnung stammen;

Identifizieren (21), durch den mindestens einen Prozessor (100) des Oszillometrie-Messsystems (10), von Parametern in den wiederholten Oszillometrie-Messwerten;

Berechnen (25), durch den mindestens einen Prozessor (100) des Oszillometrie-Messsystems (10), mindestens einer Zielfunktion aus den Parametern der wiederholten Oszillometrie-Messwerte, wobei die mindestens eine Zielfunktion Variabilität zwischen den wiederholten Oszillometrie-Messwerten erfasst,

Beurteilen (25), durch den mindestens einen Prozessor (100) des Oszillometrie-Messsystems (10), der mindestens einen Zielfunktion in Abhängigkeit von mindestens einem vorbestimmten Schwellenwert;

Akzeptieren oder Zurückweisen (26) der wiederholten Oszillometrie-Messwerte auf der Basis der Ergebnisse des Beurteilens (25) durch den mindestens einen Prozessor (100) des Oszillometrie-Messsystems (10); und

Ausgeben (C), durch den mindestens einen Prozessor (100) des Oszillometrie-Messsystems (10), von Oszillometrie-Daten anhand der Oszillometrie-Messwerte, wenn diese von dem Beurteilen (25) akzeptiert werden.

## Revendications

1. Un procédé (20, 30, 40, 50) d'acquisition de mesures d'oscillométrie avec un système de mesure d'oscillométrie (10) dans une évaluation de fonction pulmonaire, le procédé comprenant :

la réception (21) de mesures d'oscillométrie répétées, au moyen d'au moins un processeur (100) du système de mesure d'oscillométrie (10), les mesures d'oscillométrie répétées provenant d'au moins un enregistrement d'oscillométrie,

l'identification (21), au moyen du au moins un processeur (100) du système de mesure d'oscillométrie (10), de paramètres dans les mesures d'oscillométrie répétées,

le calcul (25), au moyen du au moins un processeur (100) du système de mesure d'oscillométrie (10), d'au moins une fonction objective à partir des paramètres des mesures d'oscillométrie répétées, la au moins une fonction objective capturant une variabilité entre les mesures d'oscillométrie répétées,

l'évaluation (25), au moyen du au moins un processeur du système de mesure d'oscillométrie (10), de la au moins une fonction objective sous la forme d'une fonction d'au moins un seuil prédéterminé,

en fonction des résultats de l'évaluation (25), l'acceptation ou le rejet (26), au moyen du au moins un processeur (100) du système de mesure d'oscillométrie (10), des mesures d'oscillométrie répétées, et

la production en sortie, au moyen du au moins un processeur (100) du système de mesure d'oscillométrie (10), de données d'oscillométrie au moyen des mesures d'oscillométrie si elles sont acceptées à partir de l'évaluation (25).

2. Le procédé (20, 30, 40, 50) selon la Revendication 1, où la réception (21) de mesures d'oscillométrie répétées comprend la réception de mesures d'oscillométrie répétées délimitées par des épisodes respiratoires.

3. Le procédé (20, 30, 40, 50) selon la Revendication 2, où la réception (21) de mesures d'oscillométrie délimitées par des épisodes respiratoires comprend l'isolation des épisodes respiratoires du au moins un enregistrement d'oscillométrie et où éventuellement, l'isolation des épisodes respiratoires comprend l'identification d'un marqueur dans le au moins un enregistrement d'oscillométrie comprenant au moins un élément parmi des maximas, des minimas, des passages par zéro, des passages de valeurs seuils prédéfinies de signaux de volume ou de flux.

4. Le procédé (20, 30, 40, 50) selon la Revendication 2, où la réception (21) de mesures d'oscillométrie délimitées par des épisodes respiratoires comprend la surveillance d'une respiration d'un sujet et le déclenchement d'un enregistrement des mesures d'oscillométrie après la détection d'un point souhaité dans un cycle respiratoire et où, éventuellement, le déclenchement d'un enregistrement des mesures d'oscillométrie comprend l'identification d'un marqueur dans la surveillance comprenant au moins un élément parmi des maximas, des minimas, des passages par zéro, des passages de valeurs seuils prédéfinies de signaux de volume ou de flux.

5. Le procédé (20, 30, 40, 50) selon l'une quelconque des Revendications 1 à 4, où (21) la réception de mesures d'oscillométrie répétées comprend l'enregistrement des mesures d'oscillométrie au cours du au moins un enregistrement d'oscillométrie au moyen d'un dispositif de mesure d'oscillométrie provenant du système de mesure d'oscillométrie.

**6.** Le procédé (20, 30, 40, 50) selon l'une quelconque des Revendications 1 à 5, où l'identification des paramètres dans les mesures d'oscillométrie répétées comprend l'identification d'au moins un élément parmi une résistance, une réactance ou une impédance de système respiratoire pour les mesures d'oscillométrie répétées.

**7.** Le procédé (20, 30, 40, 50) selon l'une quelconque des Revendications 1 à 6, où le calcul (25) d'au moins une fonction objective comprend le calcul d'un coefficient de variation (CV) de résistance à une fréquence unique $f^*$ en fonction de $\zeta = CV(R(f^*))$.

**8.** Le procédé (20, 30, 40, 50) selon l'une quelconque des Revendications 1 à 7, où le calcul (25) d'au moins une fonction objective comprend le calcul d'un coefficient de variation (CV) d'une impédance à une fréquence unique $f^*$ en fonction de $\zeta = CV(|Z(f^*)|)$.

**9.** Le procédé (20, 30, 40, 50) selon l'une quelconque des Revendications 1 à 8, où le calcul (25) d'au moins une fonction objective comprend le calcul d'un coefficient maximal de variation (CV) d'une résistance sur une gamme de fréquences en fonction de $\zeta = \max(CV(R(f)))$.

**10.** Le procédé (20, 30, 40, 50) selon l'une quelconque des Revendications 1 à 9, où le calcul (25) d'au moins une fonction objective comprend le calcul d'un coefficient maximal de variation (CV) d'une impédance sur une gamme de fréquences en fonction de $\zeta = \max(CV(|Z(f)|))$.

**11.** Le procédé (20, 30, 40, 50) selon l'une quelconque des Revendications 1 à 10, où le calcul (25) d'au moins une fonction objective comprend le calcul d'une moyenne de coefficients de variation (CV) d'une impédance sur $N_f$ fréquences mesurées selon

$$\zeta = \frac{1}{N_f}\sum_{i=1}^{N_f} CV\left(\left|z_{f_i}\right|\right)$$

et où, éventuellement, le calcul d'au moins une fonction objective comprend le calcul de ladite moyenne de coefficients de variation (CV) de l'impédance sur $N_f$ fréquences par l'ajout d'une fonction de pondération $W$ selon

$$\zeta = \frac{\sum_{i=1}^{N_f} W_i \cdot CV\left(\left|Z_{f_i}\right|\right)}{\sum_{i=1}^{N_f} W_i}.$$

**12.** Le procédé (20, 30, 40, 50) selon la Revendication 11, où le calcul (25) d'au moins une fonction objective comprend le calcul de ladite moyenne de coefficients de variation (CV) de l'impédance sur $N_f$ fréquences par le calcul d'une valeur quadratique moyenne selon

$$\zeta = \left(\frac{\sum_{i=1}^{N_f} W_i \cdot CV\left(\left|Z_{f_i}\right|\right)^2}{\sum_{i=1}^{N_f} W_i}\right)^{\frac{1}{2}}.$$

et/ou, où le calcul d'au moins une fonction objective comprend le calcul de ladite moyenne de coefficients de variation (CV) de l'impédance sur $N_f$ fréquences par le calcul d'une valeur quadratique moyenne dans laquelle un ordre de la racine correspond à une puissance p à laquelle chaque CV est élevé selon

$$\zeta = \left(\frac{\sum_{i=1}^{N_f} W_i \cdot CV\left(\left|Z_{f_i}\right|\right)^{\mathrm{p}}}{\sum_{i=1}^{N_f} W_i}\right)^{\frac{1}{p}}.$$

**13.** Le procédé (20, 30, 40, 50) selon l'une quelconque des Revendications 1 à 12, où le calcul (25) d'au moins une fonction objective comprend le calcul d'une somme d'écarts-types au carré divisée par une somme de moyennes au carré de |Z| sur $N_f$ fréquences mesurées selon

$$\zeta = \frac{\sum_{i=1}^{N_f} SD\left(\left|Z_{f_i}\right|\right)^2}{\sum_{i=1}^{N_f} \overline{\left|Z_{f_i}\right|}^2}.$$

**14.** Le procédé (20, 30, 40, 50) selon l'une quelconque des Revendications 1 à 13, où le calcul (25) d'au moins une fonction objective comprend le calcul d'une somme d'écarts-types au carré divisée par une somme de moyennes au carré de |Z| sur $N_f$ fréquences mesurées, avec une fonction de pondération ajoutée $W$ selon

$$\zeta = \frac{\sum_{i=1}^{N_f} W_i \cdot SD\left(\left|Z_{f_i}\right|\right)^2}{\sum_{i=1}^{N_f} W_i \cdot \overline{\left|Z_{f_i}\right|}^2}.$$

et/ou, où le calcul d'au moins une fonction objective comprend le calcul d'une somme d'écarts-types élevée à une puissance $p$ divisée par une somme de moyennes de |Z| élevée à la puissance $p$, sur $N_f$ fréquences mesurées, comprenant une fonction de pondération $W$ selon

$$\zeta = \frac{\sum_{i=1}^{N_f} W_i \cdot SD\left(\left|Z_{f_i}\right|\right)^p}{\sum_{i=1}^{N_f} W_i \cdot \overline{\left|Z_{f_i}\right|}^p}.$$

**15.** Le procédé (20, 30, 40, 50) selon l'une quelconque des Revendications 1 à 14, où le calcul (25) d'au moins une fonction objective comprend la détermination (23) si un nombre minimal $N_{min}$ de mesures d'oscillométrie est atteint avant le calcul de la au moins une fonction objective.

**16.** Le procédé (20, 30, 40, 50) selon la Revendication 15, comprenant en outre la combinaison de mesures d'oscillométrie si le nombre minimal $N_{min}$ de mesures d'oscillométrie est dépassé, la combinaison comprenant la totalité des permutations possédant au moins un nombre minimal $N_{av}$ de mesures d'oscillométrie nécessaires pour un calcul de moyennes.

**17.** Le procédé (20, 30, 40, 50) selon la Revendication 16, où l'évaluation (25) de la au moins une fonction objective comprend l'évaluation de la au moins une fonction objective au moyen d'une des permutations sélectionnée sous la forme d'une fonction du au moins un seuil prédéterminé.

**18.** Le procédé (20, 30, 40, 50) selon l'une quelconque des Revendications 15 à 17, où le calcul (25) d'au moins une fonction objective comprend le calcul de la fonction objective au moyen d'une moyenne des paramètres pour les mesures d'oscillométrie.

**19.** Un système (10) d'acquisition de mesures d'oscillométrie dans une évaluation de fonction pulmonaire, le système comprenant :

une unité de traitement (100), et
une mémoire lisible par ordinateur non transitoire couplée en communication à l'unité de traitement et comprenant des instructions de programme lisibles par ordinateur exécutables par l'unité de traitement (100) destinées à :

la réception (21) de mesures d'oscillométrie répétées, au moyen d'au moins un processeur (100) du système de mesure d'oscillométrie (10), les mesures d'oscillométrie répétées provenant d'au moins un enregistrement d'oscillométrie,
l'identification (21), au moyen du au moins un processeur (100) du système de mesure d'oscillométrie (10), de paramètres dans les mesures d'oscillométrie répétées,
le calcul (25), au moyen du au moins un processeur (100) du système de mesure d'oscillométrie (10), d'au moins une fonction objective à partir des paramètres des mesures d'oscillométrie répétées, la au moins une fonction objective capturant une variabilité entre les mesures d'oscillométrie répétées,

l'évaluation (25), au moyen du au moins un processeur (100) du système de mesure d'oscillométrie (10), de la au moins une fonction objective sous la forme d'une fonction d'au moins un seuil prédéterminé,

en fonction des résultats de l'évaluation (25), l'acceptation ou le rejet (26), au moyen du au moins un processeur (100) du système de mesure d'oscillométrie (10), des mesures d'oscillométrie répétées, et

la production en sortie (C), au moyen du au moins un processeur (100) du système de mesure d'oscillométrie (10), de données d'oscillométrie au moyen des mesures d'oscillométrie si elles sont acceptées à partir de l'évaluation (25).

**FIG. 1**

FIG. 2

30

New Test

21

Obtain Recording

22

No ← Recording/
Measurement
Technically
Valid?

Yes

23

Less ← Nmin
Valid Measurements
Obtained? → Nmin=Nav

31

No → Desired grading
reached for Nav ?

Yes

Nmin>Nav

32

Evaluate $\zeta$ for Nav of each
combination of Nmin Meas.

33

Best comb.
reaches desired
grading? → Yes

No

34

No ← Nmax
Valid Measurements
Obtained?

Yes

35

Best comb.
reaches min. acceptable
grading? → Yes

No

❌ Invalid Test

✓ Valid Test

# FIG. 3

40

21
Obtain Recording

22
Recording
Technically
Valid?

No

41
Isolate Episodes

Yes

23
Nmin
Valid Episodes
Obtained?

Nmin=Nav

Less

24
No

ζ acceptable
for Nav ?

Nmin>Nav

Yes

25
Evaluate ζ for Nav of each
combination of Nmin
Episodes

26
Best ζ
acceptable?

Yes

No

27
Nmax
Valid Episodes
Obtained?

No

Yes

(X) Invalid Test

(✓) Valid Test

FIG. 4

**FIG. 5**

FIG. 6

FIG. 7

**FIG. 8**

**EP 3 658 021 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62537228
- US 2012289852 A1
- US 6142952 A